# EUROPÄISCHE PATENTSCHRIFT

(11) **EP 2 413 907 B1**
(45) Veröffentlichungstag und Bekanntmachung des Hinweises auf die Patenterteilung: **01.10.2014**
(21) Anmeldenummer: 10711403.5
(22) Anmeldetag: 30.03.2010
(51) Int. Cl.: A61K 9/14, A61K 9/20, A61K 8/81, A23L 1/03, A61K 8/86, A23L 1/00, A23L 1/27, A23L 1/30, A61Q 19/00, A61K 47/34, A61K 9/16

(54) **VERFAHREN ZUR HERSTELLUNG VON ZUBEREITUNGEN VON IN WASSER SCHWERLÖSLICHEN SUBSTANZEN**
METHOD FOR PRODUCING PREPARATIONS OF SUBSTANCES POORLY SOLUBLE IN WATER
PROCÉDÉ DE PRODUCTION DE PRÉPARATIONS DE SUBSTANCES DIFFICILEMENT SOLUBLES DANS L'EAU

(30) Priorität: 31.03.2009 EP 09156892
(43) Veröffentlichungstag der Anmeldung: 08.02.2012
(73) Patentinhaber: BASF SE, 67056 Ludwigshafen (DE)
(72) Erfinder: MEYER-BÖHM, Kathrin, 90537 Feucht (DE); DOBRAWA, Rainer, 70469 Stuttgart (DE); FISCHER, Stefan, 67251 Freinsheim (DE); KOLTER, Karl, 67117 Limburgerhof (DE)
(86) Internationale Anmeldenummer: PCT/EP2010/054167
(87) Internationale Veröffentlichungsnummer: WO 2010/112489

(56) Entgegenhaltungen:
- EP-A1- 2 158 922
- WO-A1-2009/013202
- WO-A1-2011/032860
- WO-A1-2011/064111
- WO-A2-2007/051743
- WO-A2-2010/034688

## Beschreibung

Die vorliegende Erfindung betrifft ein Verfahren zur Herstellung von Zubereitungen von in Wasser schwer löslichen Substanzen durch Einbettung der schwerlöslichen Substanzen in Copolymeren, die durch Polymerisation von Vinylacetat und N-Vinyllactamen in Gegenwart eines Polyethers erhalten werden. Die Einbettung erfolgt durch Extrusion und, bei Temperaturen oberhalb des Schmelzpunkts der in Wasser schwerlöslichen Substanzen, wobei die Substanzen in der extrudierten Zubereitung amorph vorliegen.

Die entsprechenden Copolymere eignen sich als Solubilisatoren für die in Wasser schwerlöslichen Substanzen.

Bei der Herstellung homogener Zubereitungen insbesondere von biologisch aktiven Substanzen hat die Solubilisierung von hydrophoben, also in Wasser schwerlöslichen Stoffen, eine sehr große praktische Bedeutung erlangt.

Unter Solubilisierung ist das Löslichmachen von in einem bestimmtem Lösungsmittel, insbesondere Wasser, schwer- oder unlöslichen Substanzen durch grenzflächenaktive Verbindungen, die Solubilisatoren, zu verstehen. Solche Solubilisatoren sind in der Lage, schlecht wasserlösliche oder wasserunlösliche Stoffe in klare, höchstens opaleszierende wässrige Lösungen zu überführen, ohne dass hierbei die chemische Struktur dieser Stoffe eine Veränderung erfährt (Vgl. Römpp Chemie Lexikon, 9. Auflage, Bd.5. S. 4203, Thieme Verlag, Stuttgart, 1992).

Die hergestellten Solubilisate sind dadurch gekennzeichnet, dass der schlecht wasserlösliche oder wasserunlösliche Stoff in den Molekülassoziaten der oberflächenaktiven Verbindungen, die sich in wässriger Lösung bilden, wie beispielsweise hydrophobe Domänen oder Mizellen, kolloidal gelöst vorliegt. Die resultierenden Lösungen sind stabile oder metastabile einphasige Systeme, die optisch klar bis opaleszent erscheinen.

Solubilisatoren können beispielsweise das Aussehen von kosmetischen Formulierungen sowie von Lebensmittelzubereitungen verbessern, indem sie die Formulierungen transparent machen. Außerdem kann im Falle von pharmazeutischen Zubereitungen auch die Bioverfügbarkeit und damit die Wirkung von Arzneistoffen durch die Verwendung von Solubilisatoren gesteigert werden.

Eine weitere wünschenswerte Anforderung an Solubilisatoren ist die Fähigkeit, mit schwerlöslichen Substanzen sogenannte "feste Lösungen" auszubilden. Der Begriff "feste Lösung" bezeichnet einen Zustand, in dem eine Substanz kolloidaldispers oder im Idealfall molekulardispers in einer festen Matrix, beispielsweise einer Polymermatrix, verteilt ist. Solche festen Lösungen führen zum Beispiel bei Verwendung in festen pharmazeutischen Darreichungsformen eines schwerlöslichen Wirkstoffs zu einer verbesserten Freisetzung des Wirkstoffs. Eine wichtige Anforderung an solche feste Lösungen ist, dass sie auch bei Lagerung über längere Zeit stabil sind, d.h., dass der Wirkstoff nicht auskristallisiert. Weiterhin ist auch die Kapazität der festen Lösung, mit anderen Worten die Fähigkeit der Ausbildung von stabilen festen Lösungen mit möglichst hohen Wirkstoffgehalten, von Bedeutung.

Bei der Ausbildung von festen Lösungen spielt neben der grundsätzlichen Fähigkeit der Solubilisatoren zur Bildung von festen Lösungen auch die Hygroskopizität der Solubilisatoren eine bedeutende Rolle. Solubilisatoren, die aus der Umgebungsluft zuviel Wasser aufnehmen, führen zu einem Zerfließen der festen Lösung und der unerwünschten Kristallisation der Wirkstoffe. Auch bei der Verarbeitung zu Darreichungsformen kann eine zu große Hygroskopizität Probleme bereiten.

Viele bekannte polymere Solubilisatoren weisen die Nachteile auf, dass sie keine stabilen festen Lösungen ausbilden. Außerdem lassen sie noch Raum für Verbesserungen, was die Solubilisierung in wässrigen Systemen betrifft. Auch hinsichtlich der Verarbeitbarkeit weisen einige der bekannten Solubilisatoren aufgrund ihrer Neigung zu Klebrigkeit Nachteile auf, da sie keine ausreichend fließfähigen Pulver darstellen.

Aus der DE-A 199 350 63 sind Polyalkylenoxid-haltige Pfropfpolymere auf Basis von Vinyllactamen und Vinylacetat sowie deren Verwendung als Gashydratinhibitoren bekannt.

Aus der EP-A 953 347 ist die Verwendung von Polyalkylenoxid-haltigen Pfropfpolymerisaten als Solubilisatoren bekannt. Die dort beschriebenen Pfropfpolymerisate aus Vinylacetat und Polyalkylenoxiden stellen häufig keine Pulver sondern zähklebrige Flüssigkeiten dar, was anwendungstechnisch von Nachteil ist.

Aus der WO 2007/051743 ist die Verwendung von wasserlöslichen oder wasserdispergierbaren Copolymerisatenaus N-Vinyllactam, Vinylacetat und Polyethern, als Solubilisatoren für pharmazeutische, kosmetische, lebensmitteltechnische, agrotechnische oder sonstige technische Anwendungen bekannt. Darin wird ganz allgemein beschrieben, dass die entsprechenden Pfropfpolymerisate auch in der Schmelze mit den Wirkstoffen verarbeitet werden können.

Aus der WO 2009/013202 ist bekannt, dass solche Pfropfpolymerisate aus N-Vinyllactam, Vinylacetat und Polyethern im Extruder aufgeschmolzen und mit pulverförmigen oder flüssigen Wirkstoffen vermischt werden können, wobei die Extrusion bei Temperaturen deutlich unter dem Schmelzpunkt des Wirkstoffs beschrieben ist.

Allerdings lässt sich durch Vermischen des geschmolzenen Pfropfpolymeren mit pulverförmigen oder flüssigen Wirkstoffen keine befriedigend hohe und gleichzeitig stabile Wirkstoffbeladung erzielen. Vor allem die Erreichung eines stabilen röntgenamorphen Zustands des Wirkstoffs gelingt nicht immer in befriedigendem Maße.

Aufgabe der vorliegenden Erfindung war ein verbessertes Verfahren zur Einarbeitung schwerwasserlöslicher Substanzen in eine Formulierung mit verbesserter Löslichkeit, zu ermöglichen.

Demgemäß wurde ein Verfahren zur Herstellung von Zubereitungen von in Wasser schwerlöslichen Substanzen gefunden, wobei die schwerlöslichen Substanzen amorph in Copolymeren eingebettet vorliegen, die erhalten werden durch radikalisch initiierte Polymerisation einer Mischung aus
i) 30 bis 80 Gew.-% N-Vinyllactam,
ii) 10 bis 50 Gew.-% Vinylacetat und
iii) 10 bis 50 Gew.-% eines Polyethers,
mit der Maßgabe, dass die Summe von i), ii) und iii) gleich 100 Gew.-% ist, welches Verfahren dadurch gekennzeichnet ist, dass die die Einbettung bei Temperaturen oberhalb des Schmelzpunkts der biologisch aktiven Substanzen.

Gemäß einer Ausführungsform der Erfindung werden bevorzugte Polymerisate, erhalten aus:
i) 30 bis 70 Gew.-% N-Vinyllactam
ii) 15 bis 35 Gew.-% Vinylacetat, und
iii) 10 bis 35 Gew.-% eines Polyethers, verwendet.

Besonders bevorzugt verwendete Polymerisate sind erhältlich aus:
i) 40 bis 60 Gew.-% N-Vinyllactam
ii) 15 bis 35 Gew.-% Vinylacetat
iii) 10 bis 30 Gew.-% eines Polyethers

Ganz besonders bevorzugt verwendete Polymerisate sind erhältlich aus
i) 50 bis 60 Gew.-% N-Vinyllactam
ii) 25 bis 35 Gew.-% Vinylacetat, und
iii) 10 bis 20 Gew.-% eines Polyethers,

Auch für die bevorzugten und besonders bevorzugten Zusammensetzungen gilt die Maßgabe, dass die Summe der Komponeten i), ii), und iii) gleich 100 Gew.-% beträgt.

Als N-Vinyllactam kommen N-Vinylcaprolactam oder N-Vinylpyrrolidon oder deren Mischungen in Betracht. Bevorzugt wird N-Vinylcaprolactam verwendet.

Als Pfropfgrundlage dienen Polyether. Als Polyether kommen vorzugsweise Polyalkylenglykole in Betracht. Die Polyalkylenglykole können Molekulargewichte von 1000 bis 100000 Da [Dalton], vorzugsweise 1500 bis 35000 Da, besonders bevorzugt 1500 bis 10000 Da, aufweisen. Die Molekulargewichte werden ausgehend von der gemäß DIN 53240 gemessenen OH-Zahl bestimmt.

Als besonders bevorzugte Polyalkylenglykole kommen Polyethylenglykole in Betracht. Weiterhin eignen sich auch Polypropylenglykole, Polytetrahydrofurane oder Polybutylenglykole, die aus 2-Ethyloxiran oder 2,3-Dimethyloxiran erhalten werden.

Geeignete Polyether sind auch statistische oder blockartige Copolymere von aus Ethylenoxid, Propylenoxid und Butylenoxiden gewonnenen Polyalkylenglykolen wie beispielsweise Polyethylenglykol-Polypropylenglykol-Blockcopolymere. Die Blockcopolymere können vom AB- oder vom ABA-Typ sein.

Zu den bevorzugten Polyalkylenglykolen gehören auch solche, die an einer oder an beiden OH-Endgruppen alkyliert sind. Als Alkylreste kommen verzweigte oder unverzweigte C₁- bis C₂₂-Alkylreste in Betracht, bevorzugt C₁-C₁₈-Alkylreste, beispielsweise Methyl-, Ethyl-, n-Butyl-, Isobutyl-, Pentyl-, Hexyl-, Octyl-, Nonyl-, Decyl-, Dodecyl-, Tridecyl- oder Octadecyl-Reste.

Allgemeine Verfahren zur Herstellung der erfindungsgemäßen Pfropf-Copolymerisate sind an sich bekannt. Die Herstellung erfolgt durch freie radikalisch initiierte Polymerisation, bevorzugt in Lösung, in nichtwässrigen, organischen Lösungsmitteln oder in gemischt nichtwässrigen/wässrigen Lösungsmitteln. Geeignete Herstellverfahren sind beispielsweise in der WO 2007/051743 und der WO 2009/013202 beschrieben, auf deren Offenbarung hinsichtlich des Herstellungsverfahrens ausdrücklich Bezug genommen wird.

Vorzugsweise erfolgt die Einbettung durch Schmelzextrusion.

Die Copolymere können dem Extruder sowohl in pulverförmiger Form als auch in Form von Lösungen oder Dispersionen zugeführt werden.

Die Dispersionen oder Lösungen des Polymerisats können durch Entfernung des Dispersions- oder Lösemittels im Extruder im schmelzeflüssigen Zustand und Abkühlen der Schmelze in feste Form überführt werden.

Die so erhaltene Schmelze kann dann abgekühlt und granuliert werden. Da das Polymer im Allgemeinen wasserlöslich ist, kommen die üblichen Verfahren der Granulierung von Thermoplastschmelzen durch Abkühlen mittels Wasser weniger in Betracht. Vielmehr erfolgt ein sogenannter Heißabschlag oder Abkühlung unter Luft oder Schutzgas beispielsweise auf ein Teflon- oder Kettenband und anschließende Granulierung des erkalteten Schmelzestranges.

Die folgenden Methoden A-E können grundsätzlich verwendet werden:

| | |
|---|---|
| A | physikalische Pulvermischung aus Polymer und Wirkstoff und Zuführung dieser Pulvermischung in den Extruder |
| B | Zuführung des Wirkstoffes über einen separaten Bypass ins ungeschmolzene Polymer |
| C | Zuführung des Wirkstoffes über eine Seitendosierung ins geschmolzene Polymer (Polymer pulverförmig extrudiert) |
| D | Polymerlösung mit Wasser oder Ethylacetat mit darin dispergiertem Wirkstoff in teilentgaste Polymerschmelze; Polymer aus Lösung extrudiert |
| E | Zuführung des Wirkstoffes über einen Seitendosierung ins geschmolzene Polymer |

Für das erfindungsgemäße Verfahren eignen sich prinzipiell die üblichen, dem Fachmann bekannten Extrudertypen. Diese umfassen üblicherweise ein Gehäuse, eine Antriebseinheit mit Getriebe sowie eine Verfahrenseinheit, die aus der oder den mit den Schneckenelementen bestückten Extruderwellen besteht, wobei in diesem Falle modularer Aufbau vorausgesetzt wird.

Der Extruder besteht aus mehreren Abschnitten, die jeweils bestimmten Verfahrenseinheiten zuzuordnen sind. Jeder dieser Abschnitte besteht aus einem oder mehreren Zylindern (Zylinderblöcken) als kleinste unabhängige Einheit -und den zugehörigen Schneckenabschnitten mit den der Verfahrensaufgabe entsprechenden Schneckenelementen.

Die einzelnen Zylinder sollen beheizbar sein. Weiterhin können die Zylinder auch für eine Kühlung ausgelegt sein, beispielsweise für Kühlung mit Wasser. Bevorzugt sind die einzelnen Zylinderblöcke voneinander unabhängig heiz- und kühlbar, so dass auch längs der Extrusionsrichtung unterschiedliche Temperaturzonen eingestellt werden können

Der Extruder ist vorteilhafterweise als gleichsinnig drehender Zweischneckenextruder ausgeführt. Die Schneckenkonfiguration kann dabei je nach Produkt unterschiedlich stark scherend sein. Knetelemente müssen insbesondere in der Aufschmelzzone eingesetzt werden. Dabei können auch rückläufige Knetelemente eingesetzt werden.

Geeignete Zweischneckenextruder können einen Schneckendurchmesser von 16 bis70 mm und eine Länge von25 bis 40 D aufweisen..

Der gesamte Extruder ist ausZylinderblöcken, die einzeln temperierbar sind, aufgebaut. Die ersten zwei Zylinder können zwecks besseren Materialeinzugs temperiert sein. Ab dem dritten Zylinder wird vorzugsweise eine konstante Temperatur eingestellt, die materialspezifisch zu wählen ist und insbesondere vom Schmelzpunkt des eingesetzten Wirkstoffes und der Glasübergangstemperatur des Polymers abhängt. Die resultierende Produkttemperatur ist üblicherweise jedoch vom Schergrad des eingesetzten Schneckenelements abhängig und kann mitunter 20-30°C höher sein als die eingestellte Zylindertemperatur.

Nach der Aufschmelzzone kann eine Entgasungszone nachgeschaltet werden, die vorteilhafterweise mit Umgebungsdruck betrieben wird.

Die eingesetzten Runddüsen können einen Durchmesser von 0.5 bis 5 mm aufweisen. Andere Düsenformen, wie Schlitzdüsen können ebenfalls eingesetzt werden, vor allem dann wenn ein größerer Materialdurchsatz angestrebt wird.

Die zwei gleichläufig drehenden Schnecken sind so ausgelegt, dass nach einer Einzugszone bestehend aus Förderelementen in der dritten Heizzone schon Knetblöcke mit einer anschließenden Stauscheibe eingesetzt werden. Nach einer kurzen Entspannungszone aus Förderelementen, wird das nun geschmolzene Material erneut in einer Knetzone durchmischt. Darauf folgt eine Förderelementzone mit anschließenden Knetelementen. Es folgt eine Förderelementzone mit anschließender Knetzone. Schließlich wird der Austrag des Materials mit einer Förderelementzone sichergestellt. Die resultierenden Extrudatstränge können mit einem Granulator zu Granulatkörnern verarbeitet werden und diese können wiederum weiter zu einem Pulver zerkleinert (gemahlen) werden. Das Granulat oder Pulver kann in Kapseln gefüllt oder unter Anwendung üblicher Tablettierhilfsstoffe zu Tabletten verpresst werden.

Ferner ist es möglich, während der Extrusion Wasser, organische Lösungsmittel, Puffersubstanzen oder Weichmacher einzusetzen. Insbesondere Wasser oder flüchtige Alkohole bieten sich hierfür an. Dieses Verfahren ermöglicht die Umsetzung bei niedrigerer Temperatur. Die Lösungsmittel- bzw. Weichmachermengen liegen üblicherweise zwischen 0 und 30% der extrudierbaren Masse. Das Wasser oder Lösungsmittel kann schon durch eine Entgasungsstelle im Extruder bei Normaldruck oder durch Anlegen von Vakuum entfernt werden. Alternativ verdampfen diese Komponenten, wenn das Extrudat den Extruder verlässt und der Druck sich auf Normaldruck reduziert. Im Falle von schwerer flüchtigen Komponenten kann das Extrudat entsprechend nachgetrocknet werden.

In einer besonderen Variante des Herstellungsverfahrens wird direkt im Anschluß an die Extrusion die thermoplastische Masse zu einem tablettenähnlichen Komprimat kalandriert, das die endgültige Darreichungsform darstellt. In dieser Variante kann es sinnvoll sein, weitere Bestandteile wir z.B. Polymere zur Einstellung der Glasübergangstemperatur und der Schmelzviskosität, Sprengmittel, Solubilisatoren, Weichmacher, Farbstoffe, Geschmacksstoffe, Süßstoffe etc. schon vor oder während der Extrusion zuzusetzen. Prinzipiell können diese Stoffe auch verwendet werden, wenn das Extrudat zunächst zerkleinert und danach zu Tabletten verpresst wird.

Durch den Zusatz von kristallisationsinhibierenden Substanzen wie beispielsweise Kollidon 30 lässt sich die Stabilität der festen Lösungen erhöhen.

Weiterhin können auch zusätzlich Tenside, die die Schmelzviskosität und damit die Extrusionstemperatur herabsetzen, in die Zubereitungen eingearbeitet werden. Diese Stoffe können auch die mögliche Kristallisation positiv beeinflussen. Geeignete Stoffe sind beispielsweise Solutol® HS 15, Tween® 80, Cremophor RH40, Docusat-Natrium oder Natriumlaurylsulfat.

Die noch plastische Mischung wird vorzugsweise durch eine Düse extrudiert, abgekühlt und zerkleinert. Zur Zerkleinerung eignen sich grundsätzlich alle üblichen hierfür bekannten Techniken wie Heiss- oder Kaltabschlag.

Das Extrudat wird beispielsweise mit rotierenden Messern oder mit einem Luftstrahl abgeschlagen und anschließend mit Luft oder unter Schutzgas abgekühlt. Es ist auch möglich, das Extrudat als Schmelzestrang auf einem gekühlten Band (Edelstahl, Teflon, Kettenband) abzulegen und nach Erstarrung zu granulieren.

Anschließend kann das Extrudat gegebenenfalls gemahlen werden. Die Zubereitungen werden als riesel- und fließfähige wasserlösliche Pulver gewonnen. Bevorzugt werden Partikelgrößem von 20 bis 250 µm eingestellt.

Weiterhin ist es auch möglich die plastische Mischung aus Polymer und aktiver Substanz durch Spritzguss zu verarbeiten.

Die nach dem erfindungsgemäßen Verfahren erhaltenen Zubereitungen lassen sich grundsätzlich auf allen Gebieten einsetzen, bei denen in Wasser nur schwerlösliche oder unlösliche Substanzen entweder in wässrigen Zubereitungen zum Einsatz kommen sollen oder ihre Wirkung in wässrigem Milieu entfalten sollen.

Der Begriff "in Wasser schwerlöslich" umfasst erfindungsgemäß auch praktisch unlösliche Substanzen und bedeutet, dass für eine Lösung der Substanz in Wasser bei 20 °C mindestens 30 bis 100 g Wasser pro g Substanz benötigt wird. Bei praktisch unlöslichen Substanzen werden mindestens 10.000 g Wasser pro g Substanz benötigt.

Im Sinne der vorliegenden Erfindung sind unter in Wasser schwerlöslichen Substanzen vorzugsweise biologisch aktive Substanzen wie pharmazeutische Wirkstoffe für Mensch und Tier, kosmetische oder agrochemische Wirkstoffe oder Nahrungsergänzungsmittel oder diätetische Wirkstoffe zu verstehen.

Weiterhin kommen als zu solubilisierende schwerlösliche Substanzen auch Farbstoffe wie anorganische oder organische Pigmente in Betracht.

Als biologisch aktive Substanzen kommen erfindungsgemäß grundsätzlich alle festen Wirkstoffe in Betracht, die einen Schmelzpunkt aufweisen, der unter dem Zersetzungspunkt unter Extrusionsbedingungen der Copolymere liegt. Die Copolymere können im allgemeinen bei Temperaturen bis zu 260 °C extrudiert werden. Die Temperaturuntergrenze richtet sich nach der Zusammensetzung der zu extrudierenden Mischungen und den jeweils zu verabeitenden schwerlöslichen Substanzen.

Die verwendeten pharmazeutischen Wirkstoffe sind in Wasser unlösliche bzw. wenig lösliche Substanzen. Gemäß DAB 9 (Deutsches Arzneimittelbuch) erfolgt die Einstufung der Löslichkeit pharmazeutischer Wirkstoffe wie folgt: wenig löslich (löslich in 30 bis 100 Teilen Lösungsmittel); schwer löslich (löslich in 100 bis 1000 Teilen Lösungsmittel); praktisch unlöslich (löslich in mehr als 10000 Teilen Lösungsmittel). Die Wirkstoffe können dabei aus jedem Indikationsbereich kommen.

Als Beispiele seien hier Benzodiazepine, Antihypertensiva, Vitamine, Cytostatika - insbesondere Taxol, Anästhetika, Neuroleptika, Antidepressiva, antiviral wirksame Mittel wie beispielsweise Anti-HIV wirksame Mittel, Antibiotika, Antimykotika, Antidementiva, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel genannt.

Besonders bevorzugt sind von den oben genannten pharmazeutischen Zubereitungen solche, bei denen es sich um oral applizierbare Formulierungen handelt.

Der Gehalt an erfindungsgemäßem Solubilisator in der pharmazeutischen Zubereitung liegt, abhängig vom Wirkstoff, im Bereich von 1 bis 75 Gew.-%, bevorzugt 5 bis 60 Gew.-%, besonders bevorzugt 5 bis 50 Gew.-%.

Eine weitere besonders bevorzugte Ausführungsform bezieht sich auf pharmazeutische Zubereitungen, in denen die Wirkstoffe und das Copolymer als feste Lösung vorliegen. Dabei können die Entfernung des Lösungsmittels und die Einarbeitung der aktiven Substanz in einem Verfahrensschritt erfolgen. Hierbei beträgt das Gewichtsverhältnis von Copolymer zu Wirkstoff vorzugsweise von 1:1 bis 4:1, kann jedoch bis 100:1, insbesondere bis 15:1 betragen. Es kommt nur darauf an, dass bei Einsatz in der fertigen Arzneiform zum einen eine wirksame Menge Wirkstoff in der Arzneiform enthalten ist, und zum anderen bei oralen Arzneiformen die Formen nicht zu groß werden.

Zur Herstellung von pharmazeutischen Darreichungsfrome wie beispielsweise Tabletten können die Extrudate mit üblichen pharmazeutischen Hilfsstoffen versetzt werden.

Dabei handelt es sich um Stoffe aus der Klasse der Füllstoffe, Weichmacher, Löslichkeitsvermittler, Bindemittel, Silikate sowie Spreng- und Adsorptionsmittel, Schmiermittel, Fließmittel, Farbstoffe, Stabilisatoren wie Antioxidantien, Netzmittel, Konservierungsmittel, Formentrennmittel, Aromen oder Süßstoffe, bevorzugt um Füllstoffe, Weichmacher und Löslichkeitsvermittler.

Als Füllstoffe können z.B. anorganische Füllstoffe wie Oxide von Magnesium, Aluminium, Silicium, Titan- oder Calciumcarbonat, Calcium- oder Magnesiumphosphate oder organische Füllstoffe wie Lactose, Saccharose, Sorbit, Mannit zugesetzt werden.

Als Weichmacher eignen sich beispielsweise Triacetin, Triethylcitrat, Glycerolmonostearat, niedermolekulare Polyethylenglykole oder Poloxamere.

Als zusätzliche Löslichkeitvermittler eignen sich grenzflächenaktive Substanzen mit einem HLB-Wert (HydrophilicLipophilicBalance) größer 11, beispielsweise mit 40 Ethylenoxid-Einheitten ethoxiliertes hydriertes Ricinusöl (Cremophor® RH 40), mit 35 Ethylenoxid-Einheiten ethoxiliertes Ricinusöl (Cremophor EL), Polysorbat 80, Poloxamere oder Natriumlaurylsulfat.

Als Schmiermittel können Stearate von Aluminium, Calcium, Magnesium und Zinn, sowie Magnesiumsilikat, Silikone und ähnliche verwendet werden.

Als Fließmittel können beispielsweise Talk oder kolloidales Siliciumdioxid eingesetzt werden.

Als Bindemittel eignet sich zum Beispiel mikrokristalline Cellulose.

Als Sprengmittel können quervernetztes Polyvinylpyrrolidon oder quervernetzte Natriumcarboxymethylstärke sein. Stabilisatoren können sein Ascorbinsäure oder Tocopherol.

Farbstoffe sind z.B. Eisenoxide, Titandioxid, Triphenylmethanfarbstoffe, Azofarbstoffe, Chinolinfarbstoffe, Indigotinfarbstoffe, Carotinoide, um die Darreichungsformen einzufärben, Opakisierungsmittel wie Titandioxid oder Talkum, um die Lichtdurchlässigkeit zu erhöhen und um Farbstoffe einzusparen.

Neben der Anwendung in der Kosmetik und Pharmazie eignen sich die erfindungsgemäß hergestellten Zubereitungen auch für den Einsatz im Lebensmittelbereich, zum Beispiel für die Einarbeitung von schwer wasserlöslichen oder wasserunlösliche Nähr-, Hilfs- oder Zusatzstoffen, wie z.B. fettlösliche Vitamine oder Carotinoide. Als Beispiele seien mit Carotinoiden gefärbte Getränke genannt.

Die Anwendung der erfindungsgemäß erhaltenen Zubereitungen in der Agrochemie kann u.a. Formulierungen umfassen, die Pestizide, Herbizide, Fungizide oder Insektizide enthalten, vor allem auch solche Zubereitungen von Pflanzenschutzmitteln, die als Spritz- oder Gießbrühen zum Einsatz kommen.

Mit Hilfe des erfindungsgemäßen Verfahrens können sogenannte feste Lösungen mit schwerlöslichen Substanzen erhalten werden. Als feste Lösungen werden erfindungsgemäß Systeme bezeichnet, in denen keine kristallinen Anteile der schwerlöslichen Substanz zu beobachten sind.

Bei visueller Begutachtung der stabilen festen Lösungen sind keine amorphen Bestandteile zu erkennen. Die visuelle Begutachtung kann mit einem Lichtmikroskop sowohl mit als auch ohne Polarisationsfilter bei 40facher Vergrößerung erfolgen.

Weiterhin können die Zubereitungen auch mit Hilfe XRD (X-Ray Diffraction; Röntgendiffraktometrie) und
DSC (Differential Scanning Calorimetry) auf Kristallinität oder Amorphizität untersucht werden.

Die nach dem erfindungsgemäßen Verfahren erhaltenen Zubereitungen liegen wie gesagt amorph vor, was bedeutet, dass die kristallinen Anteile der biologisch aktiven Substanz kleiner 5 Gew.-% betragen. Vorzugsweise wird der amorphe Zustand mittels DSC oder XRD überprüft. Solch ein amorpher Zustand kann auch als röntgenamorpher Zustand bezeichnet werden.

Das erfindungsgemäße Verfahren erlaubt die Herstellung von stabilen Zubereitungen mit hoher Wirkstoffbeladung und guter Stabilität bezüglich des amorphen Zustand der schwerlöslichen Substanz.

### Beispiele

### Herstellung des Polymers

In einer Rührapparatur wurde die Vorlage ohne die Teilmenge von Zulauf 2 unter einer N₂-Atmosphäre auf 77°C aufgeheizt. Wenn die Innentemperatur von 77°C erreicht war, wurde die Teilmenge von Zulauf 2 zugegeben und 15 min anpolymerisiert. Anschließend wurden Zulauf 1 in 5h und Zulauf 2 in 2 h zudosiert. Nachdem alle Zuläufe zudosiert waren, wurde das Reaktionsgemisch noch 3h nachpolymerisiert. Nach der Nachpolymerisation wurde die Lösung auf einen Feststoffgehalt von 50 Gew.-% eingestellt.
Vorlage: 25 g Ethylacetat
   104,0 g PEG 6000,
   1,0 g von Zulauf 2
Zulauf 1: 240 g Vinylacetat
Zulauf 2: 456 g Vinylcaprolactam
   240 g Ethylacetat
Zulauf 3: 10,44 g tert-Butylperpivalat (75 gew.%-ig in Aliphatengemisch)
   67,90 g Ethylacetat

Anschliessend wurde das Lösungsmittel durch ein Srpühverfahren entfernt und ein pulverförmiges Produkt erhalten. Der K-Wert betrug 36 gemessen 1 gew.-%ig in Ethanol in.

Der Zweischneckenextruder, der für die Herstellung der in den nachfolgenden Beispielen beschriebenen Formulierungen eingesetzt wurde, wies einen Schneckendurchmesser von 16 mm und eine Länge von 40D auf. Der gesamte Extruder war aus 8 einzeln temperierbaren Zylinderblöcken aufgebaut. Die ersten zwei Zylinder wurden zwecks besseren Materialeinzugs bei 20°C bzw. bei 70°C temperiert. Ab dem dritten Zylinder wurde eine konstante Temperatur eingestellt,

Die hergestellten festen Lösungen wurden mittels XRD und DSC auf Kristallinität bzw. Amorphizität unter Verwendung folgender Geräte und Bedingungenuntersucht:

### XRD

Messgerät: Diffraktometer D 8 Advance mit 9-fach Probenwechsler (Fa.Bruker/AXS)
Messart: θ- θ Geometrie in Reflexion
Winkelbereich 2 Theta: 2-80°
Schrittweite: 0,02°
Messzeit pro Winkelschritt: 4,8s
Divergence Slit: Göbelspiegel mit 0,4 mm Steckblende
Antiscattering Slit: Sollerspalt
Detektor: Sol-X Detektor
Temperatur: Raumtemperatur
Generatoreinstellung: 40kV/50mA

### DSC

DSC Q 2000 der Fa. TA -Instruments
Parameter:
Einwaage ca. 8,5 mg
Heizrate: 20K/min
Herstellung von festen Lösungen

### Beispiel 1

1600g Polymer und 400g Fenofibrat (Schmelzpunkt 81°C) wurden in einen Turbulamischbehälter eingewogen und 10 Minuten im Turbulamischer T10B gemischt.

Die Mischung wurde unter folgenden Bedingungen extrudiert:
- Zonentemperatur ab dem 3. Zylinder: 130°C
- Schneckendrehzahl 200 UpM
- Durchsatz 1000g/h
- Düsendurchmesser 1 mm
- Düsendruck: 11 bar
- Stromaufnahme: 2,8 A
- Leistungsaufnahme: 0,3 kW

Die festen Lösungen wurden mit XRD und per DSC untersucht und als amorph befunden.

### Beispiel 2

800g Polymer und 200g Cinnarizin (Schmelzpunkt 122°C) wurden in ein Turbulamischbehälter eingewogen und 10 Minuten im Turbulamischer T10B gemischt.

Die Mischung wurde unter folgenden Bedingungen extrudiert:
- Zonentemperatur ab dem 3. Zylinder: 140°C
- Schneckendrehzahl 200 UpM
- Durchsatz 900g/h
- Düsendurchmesser 1 mm
- Materialtemperatur: 148°C
- Düsendruck: 12bar
- Stromaufnahme: 2,6 A
- Leistungsaufnahme: 0,26 kW

Die hergestellten festen Lösungen waren gemäß XRD und DSC amorph.

### Beispiel 3

800g Polymer und 200g Ketoconazol (Schmelzpunkt 146°C) wurden in ein Turbulamischbehälter eingewogen und 10 Minuten im Turbulamischer T10B gemischt.

Die Mischung wurde unteri folgenden Bedingungen extrudiert:
- Zonentemperatur ab dem 3. Zylinder: 150°C
- Schneckendrehzahl 200 UpM
- Durchsatz 900g/h
- Düsendurchmesser 1 mm
- Materialtemperatur: 155°C
- Düsendruck:10bar
- Stromaufnahme: 2,5 A
- Leistungsaufnahme: 0,24 kW

Die hergestellten festen Lösungen waren gemäß XRD und DSC amorph.

### Beispiel 4

800g Polymer und 200g Clotrimazol (Schmelzpunkt 147°C) wurden in ein Turbulamischbehälter eingewogen und 10 Minuten im Turbulamischer T10B gemischt.

Die Mischung wurde unter folgenden Bedingungen extrudiert:
- Zonentemperatur ab dem 3. Zylinder: 150°C
- Schneckendrehzahl 200 UpM
- Durchsatz 900g/h
- Düsendurchmesser 1 mm
- Materialtemperatur: 158°C
- Düsendruck: 13bar
- Stromaufnahme: 2,9 A
- Leistungsaufnahme: 0,3 kW

Die hergestellten festen Lösungen waren gemäß XRD und DSC amorph.

### Beispiel 5

800g Polymer und 200g Felodipin (Schmelzpunkt 145°C) wurden in ein Turbulamischbehälter eingewogen und 10 Minuten im Turbulamischer T10B gemischt.

Die Mischung wurde unter folgenden Bedingungen extrudiert:
- Zonentemperatur ab dem 3. Zylinder: 150°C
- Schneckendrehzahl 200 UpM
- Durchsatz 900g/h
- Düsendurchmesser 1 mm
- Materialtemperatur: 155°C
- Düsendruck: 12bar
- Stromaufnahme: 2,6 A
- Leistungsaufnahme: 0,26 kW

Die hergestellten festen Lösungen waren gemäß XRD und DSC amorph.

### Beispiel 6

1400g Polymer, 400g Fenofibrat (Schmelzpunkt 81 °C) und 200g Povidon K30 wurden in einen Turbulamischbehälter eingewogen und 10 Minuten im Turbulamischer T10B gemischt.

Die Mischung wurde unter folgenden Bedingungen extrudiert:
- Zonentemperatur ab dem 3. Zylinder: 150°C
- Schneckendrehzahl 200 UpM
- Durchsatz 1000g/h
- Düsendurchmesser 1 mm
- Düsendruck: 14bar
- Stromaufnahme: 3,2 A
- Leistungsaufnahme: 0,35 kW

Die hergestellten festen Lösungen waren gemäß XRD und DSC amorph.

### Beispiel 7

760g Polymer, 40g Natriumlaurylsulfat und 200g Felodipin (Schmelzpunkt 145°C) wurden in einen Turbulamischbehälter eingewogen und 10 Minuten im Turbulamischer T10B gemischt.

Die Mischung wurde unter folgenden Bedingungen extrudiert:
- Zonentemperatur ab dem 3. Zylinder: 160°C
- Schneckendrehzahl 200 UpM
- Durchsatz 900g/h
- Düsendurchmesser 1 mm
- Materialtemperatur: 165°C
- Düsendruck: 10bar
- Stromaufnahme: 2,4 A
- Leistungsaufnahme: 0,24 kW

Die hergestellten festen Lösungen waren gemäß XRD und DSC amorph.

## Patentansprüche

1. Verfahren zur Herstellung von Zubereitungen von in Wasser schwer löslichen Substanzen, wobei die schwerlöslichen Substanzen amorph in einem Copolymer eingebettet vorliegen, und das Copolymer erhalten wird durch radikalisch initiierte Polymerisation einer Mischung aus
i) 30 bis 80 Gew.-% N-Vinyllactam,
ii) 10 bis 50 Gew.-% Vinylacetat, und
iii) 10 bis 50 Gew.-% eines Polyethers,
mit der Maßgabe, dass die Summe der Komponenten i), ii) und iii) gleich 100 Gew.-% ist, **dadurch gekennzeichnet, dass** die Einbettung der schwerlöslichen Substanz in das Copolymer bei Temperaturen über dem Schmelzpunkt der schwerlöslichen Substanzen erfolgt, wobei in Wasser schwerlösliche Substanzen bedeutet, dass für eine Lösung der Substanz in Wasser bei 20 °C mindestens 30 bis 100 g Wasser pro g Substanz benötigt werden.

2. Verfahren nach Anspruch 1, wobei Copolymere eingesetzt werden, die aus
i) 30 bis 70 Gew.-% N-Vinyllactam,
ii) 15 bis 35 Gew.-% Vinylacetat, und
iii) 10 bis 35 Gew.-% eines Polyethers, erhalten werden.

3. Verfahren nach Anspruch 1 oder 2, wobei ein Copolymer eingesetzt wird, das unter Verwendung von N-Vinylpyrrolidon oder N-Vinylcaprolactam oder Mischungen als Komponente i) erhalten wird.

4. Verfahren nach einem der Ansprüchen 1 bis 3, wobei ein Copolymer eingesetzt wird, dass unter Verwendung von N-Vinylcaprolactam als Komponente i) erhalten wird.

5. Verfahren nach einem der Ansprüche 1 bis 4, wobei ein Copolymer eingesetzt wird, das unter Verwendung von Polyethylenglykol als Komponente ii) erhalten wird.

6. Verfahren nach einem der Ansprüche 1 bis 5, wobei Copolymere eingesetzt werden, die unter Verwendung eines Polyethylenglykols mit einem Molekulargewicht von 1000 Dalton bis 10.000 Dalton als Komponente ii) erhalten werden.

7. Verfahren nach einem der Ansprüche 1 bis 6 wobei Copolymere eingesetzt werden, die einen K-Wert von 10 bis 60 aufweisen.

8. Verfahren nach einem der Ansprüche 1 bis 6 wobei Copolymere eingesetzt werden, die einen K-Wert von 15 bis 40 aufweisen.

9. Verfahren nach einem der Ansprüche 1 bis 8, wobei es sich bei den in Wasser schwerlöslichen Substanzen um biologisch aktive Substanzen handelt.

10. Verfahren nach einem der Ansprüche 1 bis 9, zur Herstellung von pharmazeutischen Zubereitungen für die Behandlung von Krankheiten.

11. Verfahren nach einem der Ansprüche 1 bis 8 zur Herstellung von kosmetischen Zubereitungen.

12. Verfahren nach einem der Ansprüche 1 bis 8 zur Herstellung von Nahrungsergänzungsmitteln oder dietätischen Mitteln.

13. Verfahren nach einem der Ansprüche 1 bis 8 zur Herstellung von Zubereitungen von Farbstoffen.

14. Verfahren nach einem der Ansprüche 1 bis 13, **dadurch gekennzeichnet, dass** die Einbettung der schwerlöslichen Substanz in das Copolymer durch Schmelzextrusion erfolgt.

15. Verfahren nach einem der Ansprüche 1 bis 14, **dadurch gekennzeichnet, dass** die Einbettung bei Temperaturen von bis zu 260 ° C erfolgt.

16. Verfahren nach einem der Ansprüche 1 bis 15, **dadurch gekennzeichnet, dass** die Rekristallisation verhindernde Mittel zu gesetzt werden.

## Claims

1. A process for producing formulations of sparingly water-soluble substances, said sparingly soluble substances being present in amorphous embedded form in a copolymer, and said copolymer being obtained by free-radically initiated polymerization of a mixture of
i) 30 to 80% by weight of N-vinyllactam,
ii) 10 to 50% by weight of vinyl acetate, and
iii) 10 to 50% by weight of a polyether,
with the proviso that the sum of components i), ii) and iii) equals 100% by weight, which comprises embedding the sparingly soluble substance into the copolymer at temperatures above the melting point of the sparingly soluble substances, sparingly water-soluble substances meaning that, for a solution of the substance in water at 20°C, at least 30 to 100 g of water is required per g of substance.

2. The process according to claim 1, wherein copolymers obtained from
i) 30 to 70% by weight of N-vinyllactam,
ii) 15 to 35% by weight of vinyl acetate, and
iii) 10 to 35% by weight of a polyether, are used.

3. The process according to claim 1 or 2 wherein a copolymer obtained using N-vinylpyrrolidone or N-vinylcaprolactam or mixtures as component i) is used.

4. The process according to any one of claims 1 to 3, wherein a copolymer obtained using N-vinylcaprolactam as component i) is used.

5. The process according to any one of claims 1 to 4, wherein a copolymer obtained using polyethylene glycol as component ii) is used.

6. The process according to any one of claims 1 to 5, wherein copolymers obtained using a polyethylene glycol having a molecular weight of 1000 daltons to 10 000 daltons as component ii) are used.

7. The process according to any one of claims 1 to 6, wherein copolymers having a K value of 10 to 60 are used.

8. The process according to any one of claims 1 to 6, wherein copolymers having a K value of 15 to 40 are used.

9. The process according to any one of claims 1 to 8, wherein the sparingly water-soluble substances are biologically active substances.

10. The process according to any one of claims 1 to 9 for producing pharmaceutical formulations for the treatment of diseases.

11. The process according to any one of claims 1 to 8 for producing cosmetic formulations.

12. The process according to any one of claims 1 to 8 for producing food supplements or dietetic agents.

13. The process according to any one of claims 1 to 8 for producing formulations of dyes.

14. The process according to any one of claims 1 to 13, wherein the sparingly soluble substance is embedded into the copolymer by melt extrusion.

15. The process according to any one of claims 1 to 14, wherein the embedding is effected at temperatures of up to 260°C.

16. The process according to any one of claims 1 to 15, wherein agents which prevent recrystallization are added.

## Revendications

1. Procédé pour la préparation de compositions de substances peu solubles dans l'eau, les substances peu solubles se trouvant sous forme amorphe, enrobées dans un copolymère et le copolymère étant obtenu par une polymérisation initiée par voie radicalaire d'un mélange constitué par
i) 30 à 80% en poids de N-vinyllactame,
ii) 10 à 50% en poids d'acétate de vinyle et
iii) 10 à 50% en poids d'un polyéther,
à condition que la somme des composants i), ii) et iii) vaille 100% en poids, **caractérisé en ce que** l'enrobage, dans le copolymère, de la substance peu soluble est réalisé à des températures supérieures au point de fusion des substances peu solubles, l'expression substances peu solubles dans l'eau signifiant qu'il faut, pour une solution de la substance dans l'eau à 20°C, au moins 30 à 100 g d'eau par g de substance.

2. Procédé selon la revendication 1, des copolymères étant utilisés qui sont obtenus à partir de
i) 30 à 70% en poids de N-vinyllactame,
ii) 15 à 35% en poids d'acétate de vinyle et
iii) 10 à 35% en poids d'un polyéther.

3. Procédé selon la revendication 1 ou 2, un copolymère étant utilisé qui est obtenu par l'utilisation de N-vinylpyrrolidone ou de N-vinylcaprolactame ou de mélanges comme composant i).

4. Procédé selon l'une quelconque des revendications 1 à 3, un copolymère étant utilisé qui est obtenu par l'utilisation de N-vinylcaprolactame comme composant i).

5. Procédé selon l'une quelconque des revendications 1 à 4, un copolymère étant utilisé qui est obtenu par l'utilisation de polyéthylèneglycol comme composant ii).

6. Procédé selon l'une quelconque des revendications 1 à 5, des copolymères étant utilisés qui sont obtenus par l'utilisation d'un polyéthylèneglycol présentant un poids moléculaire de 1000 daltons à 10 000 daltons comme composant ii).

7. Procédé selon l'une quelconque des revendications 1 à 6, des copolymères étant utilisés qui présentent une valeur K de 10 à 60.

8. Procédé selon l'une quelconque des revendications 1 à 6, des copolymères étant utilisés qui présentent une valeur K de 15 à 40.

9. Procédé selon l'une quelconque des revendications 1 à 8, où il s'agit, pour les substances peu solubles dans l'eau, de substances biologiquement actives.

10. Procédé selon l'une quelconque des revendications 1 à 9 pour la préparation de compositions pharmaceutiques destinées au traitement de maladies.

11. Procédé selon l'une quelconque des revendications 1 à 8 pour la préparation de compositions cosmétiques.

12. Procédé selon l'une quelconque des revendications 1 à 8 pour la préparation de compléments alimentaires ou de produits diététiques.

13. Procédé selon l'une quelconque des revendications 1 à 8 pour la préparation de compositions de colorants.

14. Procédé selon l'une quelconque des revendications 1 à 13, **caractérisé en ce que** l'enrobage de la substance peu soluble dans le copolymère est réalisé par extrusion en masse fondue.

15. Procédé selon l'une quelconque des revendications 1 à 14, **caractérisé en ce que** l'enrobage a lieu à des températures allant jusqu'à 260°C.

16. Procédé selon l'une quelconque des revendications 1 à 15, **caractérisé en ce que** des agents empêchant la recristallisation sont ajoutés.
